# EUROPÄISCHE PATENTSCHRIFT

(11) **EP 3 158 956 B1**
(45) Veröffentlichungstag und Bekanntmachung des Hinweises auf die Patenterteilung: **17.07.2019**
(21) Anmeldenummer: 16002251.3
(22) Anmeldetag: 19.10.2016
(51) Int. Cl.: A61B 17/29, A61B 18/14, A61B 17/00, A61B 18/00

(54) **MIKROCHIRURGISCHES INSTRUMENT, HANDHABE UND MOTORBLOCK FÜR EIN MIKROCHIRURGISCHES INSTRUMENT**
MICROSURGICAL INSTRUMENT, HANDLE AND MOTOR BLOCK FOR A MICROSURGICAL INSTRUMENT
INSTRUMENT DE MICROCHIRURGIE, MANETTE ET BLOC-MOTEUR POUR UN INSTRUMENT DE MICROCHIRURGIE

(30) Priorität: 21.10.2015 DE 102015013923
(43) Veröffentlichungstag der Anmeldung: 26.04.2017
(73) Patentinhaber: Karl Storz SE & Co. KG, 78532 Tuttlingen (DE)
(72) Erfinder: Volkmer, Dominik, 78567 Fridingen (DE); Stefan, Jochen, 88639 Wald (DE); Thouément, Yann, 78690 Les Essarts le Roi (FR); Besse, Régis, 78280 Guyancourt (FR)
(74) Vertreter: mepat Patentanwälte

(56) Entgegenhaltungen:
- EP-A1- 1 915 966
- EP-A1- 2 837 354
- WO-A1-2013/143563
- WO-A1-2014/162495
- DE-A1- 19 722 062

## Beschreibung

Die nachfolgende Erfindung bezieht sich auf ein mikrochirurgisches Instrument und auf eine Handhabe sowie einen Motorblock für das mikrochirurgische Instrument.

Minimalinvasive Operationsmethoden sind bekannt, auch die hierfür eingesetzten Instrumente, bei denen zumindest ein Freiheitsgrad motorisch betätigt wird, was den Vorteil bietet, dass bei Wahl eines geeigneten Motors, z. B. eines Schrittmotors, sowie einer geeigneten Übersetzung sehr wohldosierte und feinfühlige Bewegungen des Arbeitseinsatzes (also des Einsatzes, mit dem gearbeitet wird) möglich sind, sodass die Auswirkungen von nicht willentlich erfolgenden Handbewegungen bzw. Zittern verringert werden können.

Gängige mikrochirurgische Instrumente, beispielsweise für die Laparoskopie, bestehen in der Regel aus einem Arbeitseinsatz mit einem Schaft und einer scheren- oder zangenartigen Arbeitsvorrichtung an dessen distalen Ende sowie einer Handhabe, über die die Freiheitsgrade der Arbeitsvorrichtung gesteuert werden. Hierbei bieten die Arbeitseinsätze beispielsweise die Freiheitsgrade Öffnen/Schließen der Branchen der Arbeitsvorrichtung, Drehung um die Längsachse sowie Verschwenken zumindest eines Längenbereichs des Schafts. Es ist möglich, einzelne oder alle Funktionen motorisch anzusteuern.

Aus EP 2 837 354 A1 ist ein mikrochirurgisches Instrument bekannt, bei dem die Verschwenkung eines distalen Schaftabschnitts mittels eines Elektromotors in der Handhabe erfolgt, der ein Kegelradgetriebe antreibt. Auf einer von dem abtriebsseitigen Kegelrad angetriebenen Sekundärwelle ist ein Stirnrad drehfest angeordnet, das in zwei Zahnstangen kämmt. Bei Drehung des Stirnrads bewegt sich dann die eine Zahnstange vor und die andere zurück. Mit den Zahnstangen ist wiederum jeweils ein Halbschaft verbunden, wobei die beiden Halbschäfte zusammen in einem Außenschaft geführt sind, der sich zu der Arbeitsvorrichtung erstreckt. Die Halbschäfte erstrecken sich bis zu dem abwinkelbaren distalen Ende und dienen als Kraftübertragungselement für die Abwinkelung.

Der Elektromotor ist dort als separates Bauteil ausgeführt und kann nur dann mit der Handhabe gekoppelt werden, wenn die Zahnräder der Motorausgangswelle und der Eingangswelle der Handhabe in einer geeigneten Winkelposition zueinander stehen. Aus diesem Grund muss zum Ankoppeln des Motors bei Bedarf der distal abwinkelbare Bereich von Hand ausgelenkt und die Zahnräder so in Eingriffsstellung gebracht werden. Sobald die Zahnräder in Eingriff stehen, erfolgt die elektrische Kontaktierung, wobei durch die Einschiebebewegung des Motorblocks Kontakte an dem Motorblock und an dem Grundkörper der Handhabe verbunden werden.

Die WO 2014/162 495 zeigt ein mikrochirurgisches Instrument, dessen Handhabe eine erste Kupplung aufweist, die zu einer zweiten Kupplung, die an einem ankoppelbaren Antrieb vorliegt, korrespondierend ausgebildet ist. Beide Kupplungen weisen Zahnungen auf, die ineinander greifen können.

Aus der WO 2013/143 563 ist eine Anordnung zur Drehmomentbegrenzung für ein elektrisches chirurgisches Werkzeug bekannt, die eine Eingangswelle und eine Abgangswelle aufweist, die miteinander verbunden sind. Die Antriebswelle stellt dazu Eingriffsmittel bereit.

Aus der EP 1 915 966 A1 sind Arretiervorrichtungen zur Verbindung von Antriebs- und Abtriebswellen offenbart.

Ausgehend von diesem Stand der Technik liegt der vorliegenden Erfindung die Aufgabe zu Grunde, ein mikrochirurgisches Instrument zu schaffen, das sich durch eine vereinfachte Kopplung von Motorblock und Handhabe auszeichnet.

Diese Aufgabe wird durch ein mikrochirurgisches Instrument mit den Merkmalen des unabhängigen Anspruchs 1 gelöst.

Ferner ergibt sich die Aufgabe, eine Handhabe zu schaffen, die auf einfache Weise mit einem Motorblock koppelbar ist und mit diesem ein mikrochirurgisches Instrument bildet.

Diese Aufgabe wird durch eine Handhabe mit den Merkmalen des Anspruchs 14 gelöst.

Schließlich ergibt sich noch die Aufgabe, einen Motorblock zu schaffen, der leicht und Zeit sparend mit einer Handhabe koppelbar ist, um ein mikrochirurgisches Instrument zu bilden.

Diese Aufgabe wird durch einen Motorblock mit den Merkmalen des Anspruchs 15 gelöst.

Bevorzugte Weiterbildungen werden durch die jeweiligen Unteransprüche beschrieben.

Das erfindungsgemäße mikrochirurgische Instrument weist eine Handhabe und einen lösbar in die Handhabe einsetzbaren Motorblock auf. Die Handhabe kann mit einem Arbeitseinsatz gekoppelt werden und weist in einer ersten Ausführungsform einen Grundkörper und eine in dem Grundkörper gelagerte Eingangswelle auf. Die Eingangswelle ist zur motorischen Betätigung zumindest eines Freiheitsgrades des Arbeitseinsatzes vorgesehen. Die Handhabe weist ferner eine Motorschnittstelle auf, in die der Motorblock einsetzbar ist, wobei an der Motorschnittstelle ein oder mehrere elektrische Kontakte und eine mechanische Kupplung vorliegen. Die mechanische Kupplung weist ein Klauenkupplungselement auf, das drehfest mit der Eingangswelle verbunden ist und mit der ein korrespondierendes, motorblockseitiges Klauenkupplungselement koppelbar ist. Darüber hinaus hat die Handhabe noch wenigstens ein Betätigungselement, mit dem ein Stromfluss durch den elektrischen Kontakt der Motorschnittstelle aktivierbar und deaktivierbar ist, um den Motorblock, wenn er in die Motorschnittstelle eingesetzt ist, zu betreiben. Erfindungsgemäß ist das Klauenkupplungselement der Handhabe auf der Eingangswelle längsverschiebbar gelagert. Dabei weist das Klauenkupplungselement der Handhabe an seinem Umfang zwei oder mehr Klauenfortsätze auf, die sich in Längsrichtung erstrecken und die in gleichen oder unterschiedlichen Winkelabständen umfänglich verteilt angeordnet sind, wobei sich die Breite der Klauenfortsätze zu ihren freien, zu der Motorschnittstelle weisenden Enden hin verjüngt.

Unter "Arbeitseinsatz", also Einsatz zum Arbeiten, wird hierin der Teil eines mikrochirurgischen Instruments verstanden, der den Schaft und die maulförmige Arbeitsvorrichtung am distalen Ende (Schere, Greifbacken, Nadelhalter o. ä.) aufweist. Der Arbeitseinsatz kann zudem ein oder mehrere Kraft- bzw. Drehmomentübertragungsmittel umfassen, die in dem Schaft verlaufen und mit denen eine Betätigungskraft von der Handhabe auf die distale Arbeitsvorrichtung ausgeübt werden kann. Der Arbeitseinsatz wird, um ein einsatzfähiges mikrochirurgisches Instrument zu erhalten, mit der Handhabe gekoppelt.

"Breite" ist hierin als umfängliche bzw. tangentiale Ausdehnung der Klauenfortsätze zu verstehen, d. h. quasi im Sinne der Zahnbreite. Die Verjüngungen dienen hierbei als Einführschrägen, die das Einführen der Klauenfortsätze des Klauenkupplungselements des anzukoppelnden Motorblocks vereinfachen.

Mit Längsrichtung ist hierbei die Längsachse der Eingangswelle gemeint, auf der das Klauenkupplungselement verschiebbar gelagert ist.

Die Branchen der Arbeitsvorrichtung können in ihren distalen Endbereichen jeweils einen Wirkabschnitt aufweisen, bevorzugt eine Schneide oder eine Greiffläche; bei dem Arbeitseinsatz kann es sich daher, je nach Ausbildung der Wirkabschnitte, beispielsweise um einen Nadelhalter, eine Fasszange, eine Schere, eine Biopsiezange, eine Spreizzange oder eine Präparierzange handeln. Es ist hierbei möglich, dass eine der Branchen feststehend und die andere Branche beweglich ist oder beide Branchen beweglich sind und schwenkbar in einer separaten Basis der Arbeitsvorrichtung gelagert sind.

Die gemäß der Erfindung vorgesehene Längsverschiebbarkeit des Klauenkupplungselements der mechanischen Kupplung der Handhabe führt dazu, dass der Motorblock nicht nur dann angekoppelt werden kann, wenn die jeweiligen Klauenfortsätze und Klauenfortsatz-Zwischenräume der Handhabe und des Motorblocks angular passend zu einander stehen, d. h. so, dass sie ineinander eintauchen können, sondern auch in den Winkelpositionen, in denen sich die Klauenfortsätze gegenseitig behindern. Das Klauenkupplungselement der Handhabe wird dann einfach beim Ankoppeln bzw. Einschieben des Motorblocks in die Motorschnittstelle um den entsprechenden Verschiebeweg, der der Eintauchtiefe der Klauenfortsätze entspricht, auf der Eingangswelle verschoben. Vorteilhaft wird der Motorblock bereits dabei mit dem elektrischen Kontakt der Motorschnittstelle verbunden, sodass dieser über das Betätigungselement der Handhabe rotiert werden kann, um die Klauenkupplungselemente der mechanischen Kupplung der Handhabe und des Motorblocks bezüglich ihres Drehwinkels so auszurichten, dass sie in Eingriff gebracht werden können. Das Klauenkupplungselement der Handhabe weist einen rotationssymmetrischen bzw. hülsenförmigen Basiskörper auf, der auf der Eingangswelle geführt wird und von dem sich die Klauenfortsätze erstrecken. "Klauenkupplungselement" ist hierin so zu verstehen, dass es sich nicht um eine vollständige Klauenkupplung handelt, sondern um einen Teil derer, der erst in Zusammenwirkung mit dem jeweils anderen Teil eine vollständige Kupplung bildet; insofern ist ein "Klauenkupplungselement" eine einzelne Kupplungsbacke.

"Motorblock" meint hierin nicht einen Motorblock im Sinne eines Grundmotors, sondern die Gesamtheit aus einem Motorgehäuse, das die Kraftleitstrukturen zur Aufnahme des eigentlichen Motors, Anschlüsse etc. aufweist, dem eigentlichen (Elektro-)Motor, Verriegelungselementen zur Anbindung an die Handhabe und eventuellen Führungskörpern zur Aufnahme in einer Führungsschiene der Motorschnittstelle der Handhabe usw. Die Definition der Breite der Klauenfortsätze entspricht hierbei der o. g. Definition.

Die Handhabe kann beispielsweise als Pistolengriff oder Pinzettengriff ausgebildet sein, während das Betätigungselement beispielsweise in Form eines Druckknopfs, Hebels o. ä. vorliegen kann.

Der Grundkörper muss im Sinne der Erfindung nicht einstückig sein, sondern kann auch aus einer Vielzahl von Bauteilen zusammengesetzt sein.

Motorblock und Handhabe in einem gekoppelten Zustand bilden das erfindungsgemäße mikrochirurgische Instrument; sowohl die erfindungsgemäße Handhabe, als auch der erfindungsgemäße Motorblock können aber getrennt gefertigt und gelagert werden. Vorteilhaft kann so das eine von beiden, das defekt ist oder aus anderen Gründen ausgetauscht werden soll, einfach gewechselt werden.

In einer weiteren Ausführungsform kann das Klauenkupplungselement der Handhabe beispielsweise drei oder mehr umfänglich verteilte Klauenfortsätze aufweisen, die sich in Längsrichtung erstrecken.

In einer noch weiteren Ausführungsform kann zwischen dem Grundkörper der Handhabe und einem der Motorschnittstelle abgewandten Ende des Klauenkupplungselements der mechanischen Kupplung der Handhabe ein Druckfederelement angeordnet sein, wobei die Einganswelle vorteilhafter Weise durch das Druckfederelement hindurch geführt sein kann, was besonders bauraumsparend ist.

Bei dem Druckfederelement kann es sich beispielsweise um eine Schraubenfeder handeln, es ist jedoch nicht ausgeschlossen, dass auch andere Druckfederelemente, wie etwa Gummiringe, Luftfedern o. ä. eingesetzt werden. Der Begriff "Ende" ist wiederum im Bezug auf die Längsrichtung der Eingangswelle zu verstehen. Das Druckfederelement hält das Klauenkupplungselement in seiner Ruhe- bzw. Koppelstellung; gegen die Federkraft wird es erst beim Ankoppeln des Motorblocks verschoben, falls die Klauenfortsätze der Klauenkupplungselemente des Motorblocks und der mechanischen Kupplung sich "überlappen" bzw. gegenseitig behindern. Damit das Klauenkupplungselement durch den Federdruck nicht von der Eingangswelle geschoben wird, kann eine Sicherung vorgesehen sein, beispielsweise eine in die Stirnfläche der Eingangswelle eingedrehte Sicherungsschraube.

Darüber hinaus kann zwischen dem Druckfederelement und dem Klauenkupplungselement eine Reibscheibe vorgesehen sein. Vorteilhafterweise ist sowohl zwischen dem Druckfederelement und dem Klauenkupplungselement als auch zwischen dem Druckfederelement und dem Grundkörper jeweils eine Reibscheibe vorgesehen. Die Reibscheibe(n) besteht/bestehen etwa aus einem Material, das in Reibpaarung mit den Oberflächen des Druckfederelements und des Klauenkupplungselements einen möglichst kleinen Gleitreibungskoeffizient aufweist, beispielsweise aus einem Kunststoff wie PTFE oder einem Polyamid.

Mit Oberflächen sind hierin zumindest die in Kontakt kommenden Oberflächen gemeint, d. h. bei der Druckfeder zumindest die obere bzw. untere Federwindung und bei dem Klauenkupplungselement die Oberfläche des der Motorschnittstelle abgewandten Endes, an der die Federkraft angreift. Über die Reibscheiben, die die Funktion von Reibungsminderungsscheiben erfüllen, soll verhindert werden, dass sich das Federelement bei Drehung der Eingangswelle sowie des darauf geführten Klauenkupplungselements unkontrolliert mitdreht und dadurch Verschleiß hervorruft. Es ist sinnvoll, zumindest zwischen dem Klauenkupplungselement und dem Federelement eine Reibscheibe vorzusehen, da hier die Relativbewegung auftritt; zudem wirkt die Reibscheibe auch als Federteller, der die Federkraft gleichmäßig in das Klauenkupplungselement einleitet.

Gemäß einer noch weiteren Ausführungsform kann die mechanische Kupplung der Handhabe ein Kupplungsgehäuse aufweisen, das mit dem Grundkörper der Handhabe verbunden ist und in dem das Klauenkupplungselement der mechanischen Kupplung der Handhabe aufgenommen ist. Das Kupplungsgehäuse weist an einem freien, der Motorschnittstelle zugewandten Ende eine Öffnung auf, durch die ein vorbestimmtes motorblockseitiges Klauenkupplungselement eingeführt werden kann, weshalb die Öffnung in Form und Abmessungen auf das vorbestimmte motorseitige Klauenkupplungselement abzustimmen ist. Ferner ist möglich, dass sich das Druckfederelement an einem der Öffnung abgewandten Ende des Kupplungsgehäuses abstützt.

Das Kupplungsgehäuse dient hierbei als Schutzvorrichtung für das Klauenkupplungselement, d. h., es handelt sich quasi um eine Kupplungsglocke. Das Kupplungsgehäuse kann über beliebige, dem Fachmann geeignet erscheinende Mittel mit dem Grundkörper verbunden sein; alternativ kann das Kupplungsgehäuse auch einstückig mit dem Grundkörper ausgebildet sein, beispielsweise als Spritzgussteil.

Das Kupplungsgehäuse kann beispielsweise eine hohlzylindrische Form haben, etwa eine im Wesentlichen kreiszylindrische Form. An seinem freien Ende kann es eine verrundete, abgeschrägte oder angefaste Außenkante aufweisen. Die Längsachse des Kupplungsgehäuses fluchtet dabei vorteilhafter Weise mit der Längsachse der Eingangswelle.

Die abgerundete, abgeschrägte oder angefaste Außenkante dient als Einführhilfe für den Motorblock, wobei beispielsweise eine Führungshülse des Motorblocks, in der das motorblockseitige Klauenkupplungselement vorliegt, passend darüber geschoben werden kann. Unter "freiem Ende" des Kupplungsgehäuses ist das Ende mit der Öffnung gemeint, d. h. die zu der Motorschnittstelle weisende Seite, von der aus das motorseitige Klauenkupplungselement eingeführt wird.

In einer weiteren Ausführungsform kann das Kupplungsgehäuse an seiner Mantelfläche zumindest eine Ausnehmung aufweisen, beispielsweise drei oder noch mehr Ausnehmungen, die über den Umfang des Kupplungsgehäuses verteilt vorliegen. Die Ausnehmungen können insbesondere in einem Bereich des Klauenkupplungselements vorliegen.

"Bereich" meint hier, dass sich darunter das Klauenkupplungselement befindet, d. h., die Ausnehmungen liegen an einer längsaxialen Position des Klauenkupplungselements vor, an der sich das Klauenkupplungselement beispielsweise in seiner Ruhe- bzw. Koppelstellung befindet.

Über die Ausnehmungen kann das Innere des Kupplungsgehäuses gereinigt werden und diese können zur Verriegelung des Motorblocks in angekoppeltem Zustand eingesetzt werden, wobei vorgesehen sein kann, dass Verriegelungselemente des Motorblocks in die Ausnehmungen eingreifen.

Gemäß einer bevorzugten Ausführungsform kann das Klauenkupplungselement der mechanischen Kupplung der Handhabe mit der Eingangswelle über eine formschlüssige Welle-Nabe-Verbindung verbunden sein. Hierbei kann die Eingangswelle ein Außenprofil aufweisen, etwa ein Keilwellenprofil, ein Polygonprofil oder eine oder mehrere Abplattungen, das oder die sich vorteilhaft entlang eines vorbestimmten Verschiebewegs des Klauenkupplungselements erstreckt/erstrecken. Das Klauenkupplungselement hat ein mit dem Außenprofil der Eingangswelle korrespondierendes Innenprofil, mit dem es in Eingriff mit dem Außenprofil steht. Über das sich in Längsrichtung erstreckende Außenprofil der Eingangswelle wird eine drehfeste Kopplung des Klauenkupplungselements mit der Eingangswelle über den kompletten Verschiebeweg ermöglicht. Die hierin genannten Profilformen sind nur Beispiele; es können darüber hinaus auch zwei oder mehr Abplattungen vorgesehen sein, die umfänglich verteilt sind. Alternativ oder zusätzlich können auch ein oder mehrere Mitnehmerelemente vorgesehen sein.

Des Weiteren kann die Handhabe ein Getriebe aufweisen, insbesondere ein Kegelradgetriebe, das ein von der Eingangswelle angetriebenes Antriebskegelrad und ein mit diesem kämmendes Abtriebskegelrad aufweist. Das Abtriebskegelrad kann eine Sekundärwelle antreiben, die bevorzugt normal zu der Eingangswelle verläuft. Die Sekundärwelle kann wiederum ein Stirnrad antreiben, mit der an gegenüberliegenden Umfangspositionen zwei Zahnstangen kämmen, die wiederum jeweils mit Kraftübertragungsmitteln verbunden sind, beispielsweise mit zwei gegeneinander verschiebbaren Halbschäften zur Abwinklung eines abwinkelbaren Schaftabschnitts eines mit der Handhabe gekoppelten Arbeitseinsatzes. Selbstverständlich können aber auch beliebige andere Funktionen des Arbeitseinsatzes über das Getriebe bedient werden, beispielsweise Öffnen/Schließen der maulförmigen Arbeitsvorrichtung oder Drehen der Arbeitsvorrichtung um die Längsachse des Schafts usw. Das Übersetzungsverhältnis kann in Abhängigkeit der gewünschten Dosierbarkeit der auszuführenden Bewegungen ausgewählt werden.

Hierzu wird auf die EP 283 734 0 A1, EP 2837 341 A1, EP 283 73 54 A1 und EP 277 75 61 A1 verwiesen.

Ferner kann der zumindest eine elektrische Kontakt in einer Buchse oder einem Stecker vorliegen, der oder die ein mit dem Grundkörper verbundenes Kontaktgehäuse und zumindest eine Kontaktzunge aufweist.

In einer noch weiteren Ausführungsform kann die Motorschnittstelle eine oder mehrere Führungsschienen aufweisen, in der zumindest ein korrespondierender Führungskörper des Motorblocks geführt werden kann. Die Führungsschiene kann vorteilhaft mit wenigstens einem Abschnitt parallel zu der Eingangswelle verlaufen, etwa in einem der mechanischen Kupplung zugewandten Endabschnitt. Über die Führungsschiene(n), die im Endabschnitt parallel zur Eingangswelle verläuft/ verlaufen, wird der Motorblock bereits beim Einführen so ausgerichtet, dass die Motorausgangswelle und die Eingangswelle der Handhabe über die jeweiligen Klauenkupplungselemente gekoppelt werden können, sodass Bedienungsfehler bei diesem Arbeitsgang weitestgehend ausgeschlossen werden.

Noch weiter kann es sich bei der Handhabe um eine Handhabe für ein elektrochirurgisches Instrument handeln, die ein elektrisches Betätigungselement aufweist, über das ein Stromfluss von einer Spannungsquelle, bevorzugt einer HF-Spannungsquelle, zu dem Arbeitseinsatz aktivierbar und deaktivierbar ist. Elektrochirurgische Werkzeuge dienen der Blutungsstillung durch Koagulation. Das elektrochirurgische Werkzeug kann ein monopolares oder bipolares Instrument sein, entsprechend kann es sich bei der erfindungsgemäßen Handhabe um eine Handhabe für beide Gattungen handeln.

Ferner kann das motorseitiges Klauenkupplungselement drehfest mit einer Motorausgangswelle verbunden sein und mit dem Klauenkupplungselement der mechanischen Kupplung gekoppelt sein, wobei in dem gekoppelten Zustand die Motorausgangswelle und die Eingangswelle fluchten.

Schließlich kann das Klauenkupplungselement des Motorblocks umfänglich verteilte Klauenfortsätze aufweisen, die sich in Längsrichtung der Motorausgangswelle erstrecken, etwa zwei, drei oder mehr Klauenfortsätze, die vorteilhaft in gleichen Winkelabständen umfänglich verteilt angeordnet sind. Bei mehreren Klauenfortsätzen können diese in unterschiedlichen Winkelabständen zueinander am Umfang vorliegen. Vorteilhafter Weise können sich die Klauenfortsätze des Klauenkupplungselements des Motorblocks in der Breite zu ihren freien, zu dem Klauenkupplungselement der mechanischen Kupplung weisenden Enden hin verjüngen. Ebenso wie die sich verjüngenden Enden des Klauenkupplungselements der mechanischen Kupplung dienen die Verjüngungen der Klauenfortsätze des Klauenkupplungselements des Motorblocks als Einführschrägen und damit einer komfortablen Ankopplung.

Die erfindungsgemäße Handhabe für ein mikrochirurgisches Instrument kann mit einem Arbeitseinsatz gekoppelt werden und weist in einer ersten Ausführungsform einen Grundkörper und eine in dem Grundkörper gelagerte Eingangswelle auf. Die Eingangswelle ist zur motorischen Betätigung zumindest eines Freiheitsgrades des Arbeitseinsatzes ausgebildet. Die Handhabe weist ferner eine Motorschnittstelle auf, in die ein Motorblock einsetzbar ist, wobei an der Motorschnittstelle ein oder mehrere elektrische Kontakte und eine mechanische Kupplung vorliegen. Die mechanische Kupplung weist ein Klauenkupplungselement auf, das drehfest mit der Eingangswelle verbunden ist und mit der ein korrespondierendes, motorblockseitiges Klauenkupplungselement koppelbar ist. Darüber hinaus hat die Handhabe noch wenigstens ein Betätigungselement, mit dem ein Stromfluss durch den elektrischen Kontakt der Motorschnittstelle aktivierbar und deaktivierbar ist, um den Motorblock, wenn er in die Motorschnittstelle eingesetzt ist, zu betreiben. Erfindungsgemäß ist das Klauenkupplungselement der Handhabe auf der Eingangswelle längsverschiebbar gelagert.

Der erfindungsgemäße Motorblock für ein mikrochirurgisches Instrument kann mechanisch und elektrisch mit der Motorschnittstelle der Handhabe gekoppelt werden und weist ein Klauenkupplungselement auf, das drehfest mit einer Motorausgangswelle verbunden ist und mit einem Klauenkupplungselement der mechanischen Kupplung der Motorschnittstelle der Handhabe gekoppelt werden kann. Ferner hat er wenigstens ein oder mehrere elektrische Verbindungselemente, die mit dem elektrischen Kontakt der Handhabe verbunden werden können. Das Klauenkupplungselement des Motorblocks weist zwei oder mehr umfänglich verteilte Klauenfortsätze auf, die sich in Längsrichtung der Motorausgangswelle erstrecken und sich in der Breite zu ihren freien Enden hin verjüngen. Bei mehreren Klauenfortsätzen auch möglich, dass diese in unterschiedlichen Winkelabständen zueinander am Umfang vorliegen.

Das elektrische Verbindungselement ist korrespondierend zu dem elektrischen Kontakt der Motorschnittstelle der Handhabe auszuwählen, beispielsweise als korrespondierendes Stecker-Buchsen-Paar. Mit dem erfindungsgemäßen Motorblock, dessen Klauenkupplungselement auch sich an ihren freien Enden verjüngende Klauenfortsätze hat, wird das Koppeln von Motorblock und Handhabe nochmals deutlich vereinfacht und beschleunigt.

Sowohl der erfindungsgemäße Motorblock als auch die erfindungsgemäße Handhabe können separat gefertigt und gelagert werden. Handhabe und Motorblock weisen dabei jeweils die Merkmale des in dem erfindungsgemäßen mikrochirurgischen Instrument eingesetzten Motorblocks und der Handhabe auf. Die erfindungsgemäß vereinfachte Koppelbarkeit von Handhabe und Motorblock ergibt sich durch ein Zusammenwirken der Merkmale des Motorblocks und der Handhabe.

Um den Motorblock mit der Handhabe zu koppeln, wird wie folgt vorgegangen:
a) in die Motorschnittstelle Einsetzen des Motorblocks,
b) in Längsrichtung der Eingangswelle in einer zu dem Klauenkupplungselement weisenden Richtung Verschieben des Motorblocks und dabei
   aa) falls die Klauenfortsätze des handhabeseitigen Klauenkupplungselements die Klauenfortsätze des motorblockseitigen Klauenkupplungselements angular überdecken,
   durch in Kontakt Bringen der Klauenfortsätze des motorblockseitigen Klauenkupplungselements und der Klauenfortsätze des handhabeseitigen Klauenkupplungselements
   - Verschieben des handhabeseitigen Klauenkupplungselements auf der Eingangswelle der Handhabe um einen vorbestimmten Verschiebeweg, der einer Eintauchtiefe der Klauenfortsätze des Klauenkupplungselements des Motorblocks in das Klauenkupplungselement der Handhabe in einer Koppelstellung entspricht, und
   - beim Einschieben des Motorblocks Verbinden der elektrischen Kontakte der Motorschnittstelle der Handhabe mit dem elektrischen Verbindungselement des Motorblocks, oderbb) falls die Klauenfortsätze des handhabeseitigen Klauenkupplungselements angular passend zu den Klauenfortsatz-Zwischenräumen des Klauenkupplungselements des Motorblocks ausgerichtet sind, entsprechend der vorbestimmten Eintauchtiefe Eintauchen der Klauenfortsätze des handhabeseitigen Klauenkupplungselements in die Klauenfortsatz-Zwischenräume des motorblockseitigen Klauenkupplungselements und umgekehrt, dabei Verbinden des elektrischen Kontakts der Handhabe mit dem elektrischen Verbindungselement des Motorblocks, danach Beenden des Verfahrens,c) nach Schritt aa) Betätigen des Betätigungselements der Handhabe, dadurch Freigeben eines Stromflusses durch den elektrischen Kontakt der Motorschnittstelle und Rotieren der Motorausgangswelle und des motorblockseitigen Klauenkupplungselements und angular passend Ausrichten der Klauenfortsätze des motorblockseitigen Klauenkupplungselements zu den Klauenfortsatz-Zwischenräumen des handhabeseitigen Klauenkupplungselements und umgekehrt,d) Zurück Verschieben des handhabeseitigen Klauenkupplungselements auf der Eingangswelle um den vorbestimmten Verschiebeweg, sodass die Klauenfortsätze des motorblockseitigen Klauenkupplungselements entlang der vorbestimmten Eintauchtiefe in die Klauenfortsatz-Zwischenräume des handhabeseitigen Klauenkupplungselements eintauchen.

"Angular überdeckend" bedeutet hierin, dass die Klauenfortsätze des Motorblocks oder der Handhabe nicht dahingehend passend ausgerichtet sind, dass sie in die jeweiligen Klauenfortsatz-Zwischenräume eintauchen können, sondern sich gegenseitig behindern. "Angular passend" meint dagegen eine zur Kopplung geeignete Winkelposition. Beim Koppeln sind grundsätzlich zwei Fälle möglich: Entweder die Klauenfortsätze des Klauenkupplungselements des Motorblocks sind schon bezüglich ihres Drehwinkels so ausgerichtet, dass deren Klauenfortsätze in die Klauenforstsatz-Zwischenräume des Klauenkupplungselements der mechanischen Kupplung eintauchen können oder nicht. Für den zweiten Fall bietet das Verfahren nun eine komfortable Möglichkeit zur Kopplung, ohne beispielsweise einen mit der Handhabe gekoppelten Arbeitseinsatz händisch auslenken zu müssen. Insofern ermöglicht das Verfahren eine komfortable, schnelle und einfache Ankopplung des Motorblocks.

Schließlich kann in dem Schritt aa) beim Verschieben des handhabeseitigen Klauenkupplungselements auf der Eingangswelle das Druckfederelement vorgespannt werden und im Schritt d) das handhabeseitige Klauenkupplungselement durch das Druckfederelement federbelastet wieder zurück verschoben werden. Hierbei gleiten die sich verjüngenden Enden der Klauenfortsätze des motorblockseitigen Klauenkupplungselements auf den sich verjüngenden Enden der Klauenfortsätze des handhabeseitigen Klauenkupplungselements und umgekehrt ab, schon bevor die Klauenfortsätze des motorblockseitigen Klauenkupplungselements und des handhabeseitigen Klauenkupplungselements angular passend zu den jeweils korrespondierenden Klauenfortsatz-Zwischenräumen ausgerichtet sind.

Diese und weitere Vorteile werden durch die nachfolgende Beschreibung unter Bezug auf die begleitenden Figuren dargelegt. Der Bezug auf die Figuren in der Beschreibung dient der Unterstützung der Beschreibung und dem erleichterten Verständnis des Gegenstands. Gegenstände oder Teile von Gegenständen, die im Wesentlichen gleich oder ähnlich sind, können mit denselben Bezugszeichen versehen sein. Die Figuren sind lediglich schematische Darstellungen von Ausführungsbeispielen der Erfindung.

Es zeigen:
- **Fig. 1**: eine perspektivische Ansicht eines Teils der Handhabe ohne Motorblock,
- **Fig. 2**: ein Längsschnitt eines Teils der Handhabe ohne Motorblock,
- **Fig. 3**: eine perspektivische Ansicht eines Teils der Handhabe mit Motorblock,
- **Fig. 4**: ein Längsschnitt eines Teils der Handhabe mit Motorblock,
- **Fig. 5**: Draufsicht eines Teils der Handhabe mit entkoppelten Klauenkupplungselementen,
- **Fig. 6**: Draufsicht eines Teils der Handhabe mit gekoppelten Klauenkupplungselementen,
- **Fig. 7**: eine perspektivische Ansicht des mikrochirurgischen Instruments.

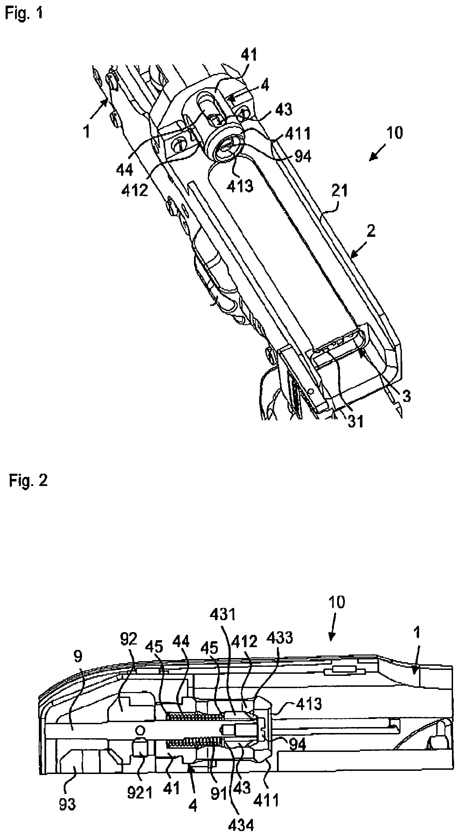

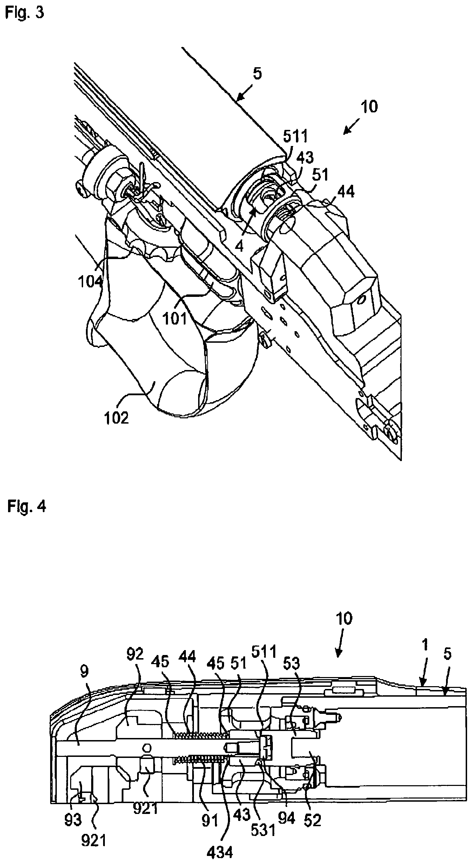

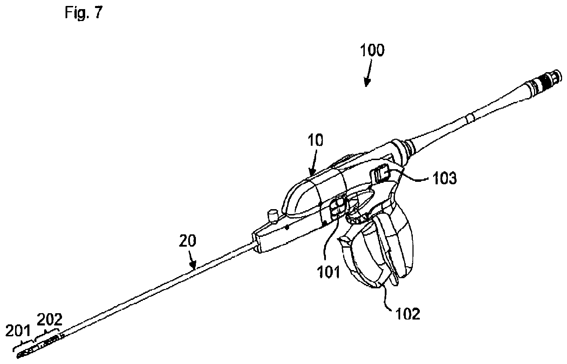

Die erfindungsgemäße Handhabe 10, die in **Fig. 1** ohne Motorblock 5 (siehe **Fig. 3** bis **Fig. 6**) dargestellt ist, ist dazu vorgesehen, mit einem Arbeitseinsatz 20 für ein mikrochirurgisches Instrument 100 gekoppelt zu werden (Siehe **Fig. 7**). Ein Arbeitseinsatz besteht hierbei aus einem Schaft, der mit der Handhabe 10 gekoppelt wird und an seinem distalen Ende bspw. eine scheren- oder zangenförmige Arbeitsvorrichtung 201 hat. Über die Handhabe 10 werden die Funktionen der distalen Arbeitsvorrichtung gesteuert, wobei die Handhabe 10 hierzu Betätigungselemente hat. Vorliegend hat die Handhabe 10 ein erstes manuelles Betätigungselement 101, mit dem die Arbeitsvorrichtung 201 um die Längsachse des Schafts rotiert werden kann.

Die erfindungsgemäße Handhabe 10 ist eine motorisierte Handhabe 10, d. h., zumindest ein Freiheitsgrad des Arbeitseinsatzes wird nicht manuell, sondern motorisch betätigt. Um den Motorblock ankoppeln zu können, hat die Handhabe 10 eine Motorschnittstelle 2, an der elektrische Kontakte 3 und eine mechanische Kupplung 4 vorliegen. Der Motorblock 5 (siehe bspw. **Fig. 3****)** wird zur Kopplung in die Führungsschiene 21 eingesetzt und in Längsrichtung vorgeschoben, bis die Kontaktzungen 31 ein korrespondierendes elektrisches Verbindungselement des Motorblocks kontaktieren und eine Motorausgangswelle mit der mechanischen Kupplung 4 verbunden ist. Die mechanische Kupplung 4 hat vorliegend ein Kupplungsgehäuse 41, das an seinem zu der Motorschnittstelle 2 weisenden Ende eine Abschrägung bzw. Anfasung 411 hat. Diese Anfasung 411 dient als Einführhilfe für einen vorbestimmten Motorblock bzw. genauer eine Führungshülse 51 (siehe **Fig. 3**), die in einer Koppelstellung über das Kupplungsgehäuse 41 geschoben wird. Auf der Mantelfläche des Kupplungsgehäuses 41 liegen umfänglich verteilte Ausnehmungen 412 vor, die der Reinigung der mechanischen Kupplung 4 dienen: Generell wäre auch möglich, dass in diesen der Motorblock verriegelt werden kann. Auch die Führungshülse 51 des Motorblocks weist Ausnehmungen 511 auf. In dem Kupplungsgehäuse 41 befindet sich ein Klauenkupplungselement 43, das drehfest mit einer Eingangswelle 9 (siehe **Fig. 2**) verbunden ist.

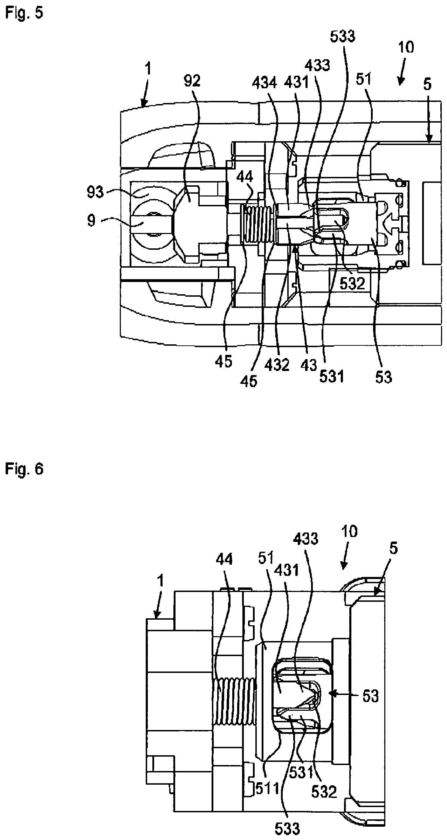

Das Klauenkupplungselement 43 ist ein hülsenförmiger Körper, der auf der Eingangswelle 9 drehfest aber längsverschiebbar geführt ist und zu der Motorschnittstelle 2 weisende, sich in Längsrichtung erstreckende Klauenfortsätze 431 (siehe **Fig. 5**) hat. Damit das Klauenkupplungselement 43 nicht von der Eingangswelle 9 rutscht, ist eine Sicherungsschraube 94 vorgesehen, deren Kopfdurchmesser größer ist als der Innendurchmesser des Klauenkupplungselements 43. Die Verbindung des Klauenkupplungselements 43 und der Eingangswelle 9 ist über eine formschlüssige Welle-Nabe-Verbindung, nämlich eine Abplattung 91 auf dem Außenquerschnitt der Eingangswelle 9 und eine korrespondierende Innenform an dem Innenquerschnitt des Klauenkupplungselements 43 realisiert. Damit das Klauenkupplungselement 43 in Längsrichtung der Eingangswelle 9 verschoben werden kann, erstreckt sich die Abplattung 91 über eine vorbestimmte Länge, die den möglichen Verschiebeweg vorgibt und begrenzt.

Zusammen mit einem korrespondierenden Klauenkupplungselement 53 des Motorblocks 5 (siehe **Fig. 4**) ergibt sich dann eine vollständige Klauenkupplung, über die das Motormoment auf die Eingangswelle 9 übertragen werden kann. Das korrespondierende Klauenkupplungselement 53 des Motorblocks 5 kann durch die Öffnung 413 des Kupplungsgehäuses 41 (siehe **Fig. 1**) eingeführt und mit dem Klauenkupplungselement 43 in Eingriff gebracht werden.

Zwischen einem den sich verjüngenden Enden 433 der Klauenfortsätze 431 des Klauenkupplungselements 43 abgewandten Ende 434 des Klauenkupplungselements 43 und einem der Öffnung 413 abgewandten Ende des Kupplungsgehäuses 41 ist eine Druckfeder 44 angeordnet, durch die die Eingangswelle 9 geführt ist, wie in **Fig. 2** und **Fig. 4** zu sehen ist. Die Druckfeder 44 stützt sich einenends an dem der Öffnung 413 abgewandten Ende des Kupplungsgehäuses 41 und anderenends an dem Ende 434 des Klauenkupplungselements 43 ab. Über die Druckfeder 44 wird eine längsaxiale Ruhestellung des Klauenkupplungselements 43 vorgegeben, die der Koppelstellung mit dem korrespondierenden Klauenkupplungselement 53 des Motorblocks 5 (siehe **Fig. 4**) entspricht. Da das Klauenkupplungselement 43 sowie die Eingangswelle 9 bezüglich des Grundkörpers 1, des Kupplungsgehäuses 41 sowie der Druckfeder 44 bewegte Teile sind, sind zwischen einem zu dem Klauenkupplungselement 43 weisenden Ende der Druckfeder 44 und einem zu dem Ende des Getriebegehäuses 41, das der Öffnung 413 abgewandt ist, Reibscheiben 45 vorgesehen. Diese Reibscheiben 45 dienen der Reibungsminderung und bestehen aus einem Material bzw. haben eine Oberflächenbeschaffenheit, das bzw. die in Reibpaarung mit den Oberflächen der Druckfeder 44 und des Klauenkupplungselements 43 einen möglichst geringen Gleitreibungskoeffizienten hat, beispielsweise ein Kunststoff wie ein Polyamid oder PTFE.

Die Eingangswelle 9 ist in dem Grundkörper 1 der Handhabe 10 in geeigneter Weise gelagert, etwa über eine Gleitlagerung. Wie in **Fig. 2** und **Fig. 4** dargestellt, ist ferner auf der Eingangswelle 9 ein antriebsseitiges Kegelrad 92 über den Gewindestift 921 drehfest befestigt, das ein normal dazu stehendes abtriebsseitiges Kegelrad 93 antreibt, das wiederum drehfest auf einer Sekundärwelle befestigt ist.

Mit der erfindungsgemäßen Handhabe 10 kann aufgrund der Ausrichtung der Sekundärwelle normal zur Längsrichtung des Schafts eines Arbeitseinsatzes über den motorischen Antrieb besonders vorteilhaft eine Abknickbewegung des Schafts des Arbeitseinsatzes erreicht werden, dies ist jedoch figurativ nicht gezeigt. Beispielsweise kann an einem dem abtriebsseitigen Kegelrad 93 abgewandten Ende der Sekundärwelle ein Stirnrad vorgesehen sein, das an zwei gegenüberliegenden Umfangspositionen mit zwei in Längsrichtung des Schafts des Arbeitseinsatzes ausgerichteten Zahnstangen kämmt. Über diese Zahnstangen, die sich bei einer Drehbewegung des Stirnrads gegenläufig bewegen, können dann beispielsweise Halbschäfte angetrieben werden, die in einem Außenschaft des Arbeitseinsatzes geführt sind und die Betätigungskräfte zu einem abknickbaren Schaftabschnitt übertragen.

In den **Fig. 3** und **Fig. 4** ist die Handhabe 10 mit angekoppeltem Motorblock 5 gezeigt. Bei der gezeigten Handhabe 10 handelt es sich um eine Handhabe 10 mit als Betätigungselement ausgebildetem Pistolengriff 102, die neben dem ersten manuellen Betätigungselement 101 zum Rotieren der Arbeitsvorrichtung um die Längsachse ein weiteres manuelles Betätigungselement 104 hat. Die Führungshülse 51 des Motorblocks 5 ist über das Kupplungsgehäuse 41 geschoben, sodass dieser bezüglich der mechanischen Kupplung 4 der Handhabe 10 räumlich festgelegt ist. In **Fig. 4** ist jedoch das Kupplungsgehäuse 41 (siehe **Fig. 1** und **Fig. 2**) zur besseren Sichtbarkeit der darin aufgenommenen Bauteile ausgeblendet. Das Klauenkupplungselement 53 des Motorblocks 5 ist drehfest mit der Motorausgangswelle 52 verbunden und hat umfänglich verteilte Klauenfortsätze 531, die in der Koppelstellung in die Klauenfortsatz-Zwischenräume des Klauenkupplungselements 43 eintauchen und so die formschlüssige Verbindung zur Drehmomentübertragung herstellen. Die Druckfeder 44 ist gelängt, da sich die Kupplung in Koppelstellung befindet, und das handhabeseitige Klauenkupplungselement 43 liegt an der Sicherungsschraube 94 an.

Beim Koppeln des Motorblocks 5 mit dem Grundkörper 1 der Handhabe 10 können zwei Fälle auftreten: Entweder die Klauenkupplungselemente 43, 53 der mechanischen Kupplung 4 und des Motorblocks 5 sind dahingehend passend zueinander orientiert, dass die jeweiligen Klauenfortsätze 431, 531 in die korrespondierenden Klauenfortsatz-Zwischenräume 432,532 eintauchen können, oder sie sind nicht passend orientiert. Im ersten Fall werden die Klauenfortsätze 531 des Motorblocks 5 beim Einschieben des Motorblocks in die Motorschnittstelle 2 bzw. genauer zwischen den Führungsschienen 21 (siehe **Fig. 1**) in die Klauenfortsatz-Zwischenräume 432 des Klauenkupplungselements 43 der mechanischen Kupplung 4 der Handhabe 10 eintauchen, im anderen Fall ist dies nicht möglich, da sich die jeweiligen Klauenfortsätze 431, 531 überdecken, d. h. behindern; dies ist in **Fig. 5** dargestellt.

Bisher war es nicht möglich, die Motorausgangswelle 52 durch Betätigung des Betätigungselements zu rotieren, da die elektrische Kopplung des Motorblocks 5 erst in der Koppelstellung möglich war, d. h., wenn die Klauenfortsätze 531 des motorblockseitigen Klauenkupplungselements 53 in die Klauenfortsatz-Zwischenräume 432 des Klauenkupplungselements 43 der mechanischen Kupplung 4 eingreifen. In der Regel weist der Motorblock 5 zudem selbst ein Getriebe mit einem sehr hohen Übersetzungsverhältnis auf, eventuell sogar eine Schneckenradstufe, sodass die Motorausgangswelle aufgrund von Selbsthemmung auch nicht händisch gedreht werden kann.

Bisher musste also, um die Klauenkupplungselemente 43,53 zu koppeln, der Arbeitseinsatz an seinem abknickbaren Ende ausgelenkt werden, um mittelbar die Eingangswelle 9 zu rotieren und die beiden Klauenkupplungselemente 43,53 bezüglich ihres Drehwinkels so zueinander auszurichten, dass diese in Eingriff gebracht werden können; dies ist jedoch nicht nur umständlich, sondern hat auch den Nachteil, dass die sterile Arbeitsvorrichtung angefasst werden muss.

Die erfindungsgemäße Handhabe 10 erlaubt es, dass der Motorblock 5 schon elektrisch verbunden ist, während die Klauenkupplungselemente 43,53 noch nicht in Eingriff stehen. Wenn die Klauenfortsätze 431,531 beim Einsetzen des Motorblocks 5 zueinander hinsichtlich ihres Drehwinkels überdeckend ausgerichtet sind, wird das handhabeseitige Klauenkupplungselement 43 durch das motorblockseitige Klauenkupplungselement 53, bzw. genauer durch Kontakt der jeweiligen Klauenfortsätze 431,531, in längsaxialer Richtung verschoben, sodass die Druckfeder 44 zusammengedrückt wird. In dieser Stellung sind nun die elektrischen Kontakte 3 mit dem korrespondierenden elektrischen Anschluss des Motorblocks 5 verbunden, sodass die Motorausgangswelle 52 einfach durch Betätigung des Betätigungselements, z. B. ein einfacher Schalter, rotiert werden kann.

In der Folge beginnen die sich verjüngenden Enden 433,533 der Klauenkupplungselemente 43,53 auf einander abzugleiten, was letztlich, sobald die Klauenfortsätze 531 des motorblockseitigen Klauenkupplungselements 53 passend zu den Klauenfortsatz-Zwischenräumen 432 des Klauenkupplungselements der mechanischen Kupplung 4 ausgerichtet sind, dazu führt, dass die Klauenfortsätze 531 des motorblockseitigen Klauenkupplungselements 53 in die Klauenfortsatz-Zwischenräume 432 der mechanischen Kupplung 4 eintauchen. Das Klauenkupplungselement 43 der mechanischen Kupplung 4 wird hierbei durch die vorgespannte Druckfeder 44 zurück in seine Ruhestellung geschoben; dieser Vorgang wird durch die Darstellung der **Fig. 6** verdeutlicht.

Vorteilhaft entfällt unter Verwendung der erfindungsgemäßen Handhabe 10 bei der Ankopplung des Motorblocks 5 der Schritt, dass der distale abknickbare Schafabschnitt des Arbeitseinsatzes zum Ankoppeln des Motorblocks 5 ausgelenkt werden muss. Hierdurch wird der Zeitaufwand zum Ankoppeln beträchtlich reduziert und die Gefahr von Kontaminationen des Arbeitseinsatzes reduziert. Die erfindungsgemäße Handhabe erlaubt es daher, die Arbeitseffizienz des Chirurgen zu erhöhen und hilft postoperative Komplikationen durch Infektionen zu vermeiden.

In **Fig. 7** ist schließlich eine Übersicht des erfindungsgemäßen mikrochirurgischen Instruments 100 gezeigt. Das mikrochirurgische Instrument 100 besteht im Wesentlichen aus einer Handhabe 10 und dem angekoppelten Arbeitseinsatz 20, der wiederum aus Arbeitsvorrichtung 201 und verbindendem Schaft besteht.

Über die Handhabe 10, bzw. genauer, deren Betätigungselemente 101,102,103 lassen sich die Funktionen des Arbeitseinsatzes 20 steuern. Vorliegend handelt es sich bei den Funktionen um: Drehen der Arbeitsvorrichtung 201 um die Längsachse des Schafts, Öffnen/Schließen der Branchen und Abwinkeln des abwinkelbaren Schaftabschnitts 202. Die Handhabe 10 hat drei Betätigungselemente 101,102,103, wobei über das erste (manuelle) Betätigungselement 101 die Arbeitsvorrichtung 201 um die Längsachse rotiert werden kann, über das zweite (manuelle) Betätigungselement 102 die Branchen geöffnet und geschlossen werden können und über das Betätigungselement 103, das ein elektrisches Betätigungselement ist, die Stromzufuhr zum Motorblock aktiviert werden kann.

### BEZUGSZEICHENLISTE

- 100: Mikrochirurgisches Instrument
- 10: Handhabe
- 101: Erstes manuelles Betätigungselement
- 102: Zweites manuelles Betätigungselement, Pistolengriff
- 103: Elektrisches Betätigungselement/Schalter
- 104: weiteres Betätigungselement
- 20: Arbeitseinsatz
- 201: Arbeitsvorrichtung
- 202: Abwinkelbarer Schaftabschnitt
- 1: Grundkörper der Handhabe
- 2: Motorschnittstelle
- 21: Führungsschiene
- 3: Elektrische Kontakte
- 31: Kontaktzungen
- 4: Mechanische Kupplung
- 41: Kupplungsgehäuse
- 411: Anfasung bzw. Abrundung des Kupplungsgehäuses
- 412: Ausnehmungen des Kupplungsgehäuses
- 413: Öffnung des Kupplungsgehäuses
- 43: Klauenkupplungselement
- 431: Klauenfortsätze des Klauenkupplungselements
- 432: Klauenfortsatz-Zwischenräume des Klauenkupplungselements
- 433: Sich verjüngende Enden der Klauenfortsätze
- 434: Den sich verjüngenden Enden abgewandtes Ende des Klauenkupplungselements
- 44: Druckfederelement
- 45: Reibscheiben
- 5: Motorblock
- 51: Führungshülse des Motorblocks
- 511: Ausnehmungen der Führungshülse
- 52: Ausgangswelle des Motorblocks
- 53: Klauenkupplungselement des Motorblocks
- 531: Klauenfortsätze des Klauenkupplungselement des Motorblocks
- 532: Klauenfortsatz-Zwischenräume
- 533: Sich verjüngende Enden der Klauenfortsätze
- 9: Eingangswelle der Handhabe
- 91: Längsverlaufende Abplattung der Eingangswelle
- 92: Antriebsseitiges Kegelrad
- 921: Gewindestift
- 93: Abtriebsseitiges Kegelrad
- 94: Sicherungsschraube

## Patentansprüche

1. Mikrochirurgisches Instrument (100), das eine Handhabe (10) und einen lösbar einsetzbaren Motorblock (5) aufweist,
wobei die Handhabe (10) mit einem Arbeitseinsatz (20) koppelbar ist und aufweist:
- einen Grundkörper (1),
- eine in dem Grundkörper (1) gelagerte Eingangswelle (9), zur motorischen Betätigung zumindest eines Freiheitsgrades des Arbeitseinsatzes (20),
- eine Motorschnittstelle (2), in die der Motorblock (5) einsetzbar ist, und an der zumindest ein elektrischer Kontakt (3) und eine mechanische Kupplung (4) vorliegen, wobei die mechanische Kupplung (4) ein Klauenkupplungselement (43) aufweist, das drehfest mit der Eingangswelle (9) verbunden ist und mit einem korrespondierenden motorblockseitigen Klauenkupplungselement (53) koppelbar ist,
- zumindest ein Betätigungselement, mit dem ein Stromfluss durch den elektrischen Kontakt (3) der Motorschnittstelle (2) aktivierbar und deaktivierbar ist,
**dadurch gekennzeichnet, dass**
das Klauenkupplungselement (43) der Handhabe (10) auf der Eingangswelle (9) längsverschiebbar gelagert ist, wobei das Klauenkupplungselement (43) der Handhabe (10) an seinem Umfang zumindest zwei Klauenfortsätze (431) aufweist, die sich in Längsrichtung erstrecken, die in gleichen oder unterschiedlichen Winkelabständen umfänglich verteilt angeordnet sind, wobei sich die Breite der Klauenfortsätze (431) zu ihren freien, zu der Motorschnittstelle (2) weisenden Enden (433) hin verjüngt.

2. Mikrochirurgisches Instrument (100) nach Anspruch 1,
**dadurch gekennzeichnet, dass**
das Klauenkupplungselement (43) der Handhabe (10) an seinem Umfang bevorzugt drei oder mehr der umfänglich verteilten Klauenfortsätze (431) aufweist.

3. Mikrochirurgisches Instrument (100) nach Anspruch 1 oder 2,
**dadurch gekennzeichnet, dass**
zwischen dem Grundkörper (1) der Handhabe (10) und dem von der Motorschnittstelle (2) abgewandten Ende des Klauenkupplungselements (43) der mechanischen Kupplung (4) der Handhabe (10) ein Druckfederelement (44) angeordnet ist, wobei bevorzugt die Eingangswelle (9) durch das Druckfederelement (44) hindurch geführt ist.

4. Mikrochirurgisches Instrument (100) nach Anspruch 3,
**dadurch gekennzeichnet, dass**
zwischen dem Druckfederelement (44) und dem Klauenkupplungselement (43) eine Reibscheibe (45) oder zwischen dem Druckfederelement (44) und dem Klauenkupplungselement (43) und zwischen dem Druckfederelement (44) und dem Grundkörper (1) jeweils eine Reibscheibe (45) angeordnet ist, die bevorzugt aus einem Material besteht, das in Reibpaarung mit den Oberflächen des Druckfederelements (44) und des Klauenkupplungselements (43) einen kleinen Gleitreibungskoeffizient aufweist und besonders bevorzugt aus einem Kunststoff besteht.

5. Mikrochirurgisches Instrument (100) nach zumindest einem der Ansprüche 3 oder 4,
**dadurch gekennzeichnet, dass**
die mechanische Kupplung (4) ein Kupplungsgehäuse (41) aufweist, das mit dem Grundkörper (1) verbunden ist und in dem das Klauenkupplungselement (43) aufgenommen ist,
- wobei das Kupplungsgehäuse (41) an einem freien, der Motorschnittstelle (2) zugewandten Ende eine Öffnung (413) aufweist, durch die ein vorgegebenes motorblockseitiges Klauenkupplungselement (52) führbar ist, und
- wobei sich bevorzugt das Druckfederelement (44) an einem der Öffnung (413) abgewandten Ende des Kupplungsgehäuses (41) abstützt.

6. Mikrochirurgisches Instrument (100) nach Anspruch 5,
**dadurch gekennzeichnet, dass**
- das Kupplungsgehäuse (41) eine hohlzylindrische Form hat, bevorzugt eine im Wesentlichen kreiszylindrische Form, und besonders bevorzugt an dem freien Ende zumindest eine verrundete, abgeschrägte oder angefaste Außenkante (411) aufweist,
- und wobei eine Längsachse des Kupplungsgehäuses (41) und eine Längsachse der Eingangswelle (9) fluchten.

7. Mikrochirurgisches Instrument (100) nach Anspruch 6,
**dadurch gekennzeichnet, dass**
das Kupplungsgehäuse (41) an seiner Mantelfläche zumindest eine Ausnehmung (412) aufweist, bevorzugt drei oder mehr Ausnehmungen (412), die über den Umfang des Kupplungsgehäuses (41) verteilt vorliegen, wobei die Ausnehmungen (412) bevorzugt in einem Bereich des Klauenkupplungselements (43) vorliegen.

8. Mikrochirurgisches Instrument (100) nach zumindest einem der Ansprüche 1 bis 7,
**dadurch gekennzeichnet, dass**
- das Klauenkupplungselement (43) der mechanischen Kupplung (4) der Handhabe (10) mit der Eingangswelle (9) über eine formschlüssige Welle-Nabe-Verbindung verbunden ist,
- wobei bevorzugt die Eingangswelle (9) ein Außenprofil, bevorzugt ein Keilwellenprofil, ein Polygonprofil oder eine oder mehr Abplattung(en) (91) aufweist, die/das sich besonders bevorzugt entlang eines vorbestimmten Verschiebewegs des Klauenkupplungselements (43) erstreckt, und das Klauenkupplungselement (43) ein mit dem Außenprofil der Eingangswelle (9) korrespondierendes Innenprofil aufweist.

9. Mikrochirurgisches Instrument (100) nach zumindest einem der Ansprüche 1 bis 8,
**dadurch gekennzeichnet, dass**
- die Handhabe (10) ein Getriebe aufweist, bevorzugt ein Kegelradgetriebe, das ein von der Eingangswelle (9) angetriebenes Antriebskegelrad (92) und ein mit diesem kämmendes Abtriebskegelrad (93) aufweist, das eine Sekundärwelle antreibt, die bevorzugt normal zu der Eingangswelle (9) verläuft,
und/oder
- der zumindest eine elektrische Kontakt (3) in einer Buchse oder Stecker vorliegt, die/der ein Kontaktgehäuse und zumindest eine Kontaktzunge (31) aufweist, wobei das Kontaktgehäuse mit dem Grundkörper (1) verbunden ist.

10. Mikrochirurgisches Instrument (100) nach zumindest einem der Ansprüche 1 bis 9,
**dadurch gekennzeichnet, dass**
die Motorschnittstelle (2) zumindest eine Führungsschiene (21) aufweist, in der zumindest ein korrespondierender Führungskörper des Motorblocks (5) geführt wird, wobei die Führungsschiene (21) bevorzugt in zumindest einem Abschnitt parallel zu der Eingangswelle (9) verläuft, besonders bevorzugt in einem der mechanischen Kupplung (4) zugewandten Endabschnitt.

11. Mikrochirurgisches Instrument (100) nach zumindest einem der Ansprüche 1 bis 10,
**dadurch gekennzeichnet, dass**
das mikrochirurgische Instrument (100) ein elektrochirurgisches mikrochirurgisches Instrument (100) ist und die Handhabe (10) ein elektrisches Betätigungselement aufweist, über das ein Stromfluss von einer Spannungsquelle, bevorzugt einer HF-Spannungsquelle, zu dem Arbeitseinsatz (20) aktivierbar und deaktivierbar ist.

12. Mikrochirurgisches Instrument (100) nach zumindest einem der Ansprüche 1 bis 11,
**dadurch gekennzeichnet, dass**
das motorseitiges Klauenkupplungselement (53) drehfest mit einer Motorausgangswelle (52) verbunden ist und mit dem Klauenkupplungselement (43) der mechanischen Kupplung (4) gekoppelt ist, wobei die Motorausgangswelle (52) des Motorblocks (5) und die Eingangswelle (9) der Handhabe (10) im gekoppeltem Zustand fluchten.

13. Mikrochirurgisches Instrument (100) nach Anspruch 12,
**dadurch gekennzeichnet, dass**
das Klauenkupplungselement (53) des Motorblocks (5) an seinem Umfang zumindest zwei Klauenfortsätze (531), bevorzugt drei oder mehr umfänglich verteilte Klauenfortsätze (531) aufweist, die sich in Längsrichtung der Motorausgangswelle (52) erstrecken, die in gleichen oder unterschiedlichen Winkelabständen umfänglich verteilt angeordnet sind, wobei sich die Klauenfortsätze (531) in der Breite zu ihren freien, zu dem Klauenkupplungselement (43) der mechanischen Kupplung (4) weisenden Enden (533) hin verjüngen.

14. Handhabe (10) für ein mikrochirurgisches Instrument nach zumindest einem der Ansprüche 1 bis 13, das einen Motorblock (5) aufweist,
wobei die Handhabe (10) mit einem Arbeitseinsatz (20) koppelbar ist und aufweist:
- einen Grundkörper (1) und
- eine in dem Grundkörper (1) gelagerte Eingangswelle (9), die zur motorischen Betätigung zumindest eines Freiheitsgrades des Arbeitseinsatzes (20) ausgebildet ist, und
- eine Motorschnittstelle (2), in die ein Motorblock (5) einsetzbar ist, wobei an der Motorschnittstelle (2) zumindest ein elektrischer Kontakt (3) und eine mechanische Kupplung (4) vorliegen, wobei die mechanische Kupplung (4) ein Klauenkupplungselement (43) aufweist, das drehfest mit der Eingangswelle (9) verbunden ist und mit dem ein korrespondierendes motorblockseitiges Klauenkupplungselement (53) koppelbar ist, und
- zumindest ein Betätigungselement, mit dem ein Stromfluss durch den elektrischen Kontakt (3) der Motorschnittstelle (2) aktivierbar und deaktivierbar ist,
**dadurch gekennzeichnet, dass**
das Klauenkupplungselement (43) der Handhabe (10) auf der Eingangswelle (9) längsverschiebbar gelagert ist, wobei das Klauenkupplungselement (53) des Motorblocks (5) zumindest zwei umfänglich verteilte Klauenfortsätze (531) aufweist, die sich in Längsrichtung der Motorausgangswelle (52) erstrecken und deren Breite sich zu ihren freien Enden (533) hin verjüngt.

15. Motorblock (5) für ein mikrochirurgisches Instrument (100) nach zumindest einem der Ansprüche 1 bis 13, wobei der Motorblock (5)
- mechanisch und elektrisch mit der Motorschnittstelle (2) der Handhabe (10) koppelbar ist, und
- zumindest ein elektrisches Verbindungselement aufweist, das mit dem elektrischen Kontakt (3) der Handhabe (10) verbindbar ist, und
- ein Klauenkupplungselement (53) aufweist, das drehfest mit einer Motorausgangswelle (52) verbunden ist und mit einem Klauenkupplungselement (43) der mechanischen Kupplung (4) der Motorschnittstelle (2) der Handhabe (10) koppelbar ist, wobei das Klauenkupplungselement (53) des Motorblocks (5) zumindest zwei umfänglich verteilte Klauenfortsätze (531) aufweist, die sich in Längsrichtung der Motorausgangswelle (52) erstrecken und deren Breite sich zu ihren freien Enden (533) hin verjüngt.

## Claims

1. A microsurgical instrument (100) comprising a handle (10) and a removably insertable engine block (5),
wherein the handle (10) can be coupled with a work piece (20) and comprises:
- a base body (1),
- an input shaft (9) mounted in the base body (1), for motorized actuation of at least one degree of freedom of the work piece (20),
- an engine interface (2), into which the engine block (5) can be installed, and on which at least one electric contact (3) and one mechanical coupling (4) are situated, wherein the mechanical coupling (4) comprises a claw coupling element (43) that is connected non-rotatably with the input shaft (9) and can be coupled with a corresponding claw coupling element (53) of the engine block,
- at least one actuation element, with which a current flow can be activated and deactivated by the electric contact (3) of the engine interface (2),
**characterized in that**
the claw coupling element (43) of the handle (10) is mounted so that it can be slid lengthwise on the input shaft (9), wherein the claw coupling element (43) of the handle (10) comprises on its periphery at least two claw extensions (431) extending in the longitudinal direction, which are peripherally distributed at equal or different angle distances, wherein the thickness of the claw extensions (431) tapers toward their free ends (433) pointing to the engine interface (2).

2. A microsurgical instrument (100) according to claim 1,
**characterized in that**
the claw coupling element (43) of the handle (10) comprises on its periphery preferably three or more of the peripherally distributed claw extensions (431).

3. A microsurgical instrument (100) according to claim 1 or 2,
**characterized in that**
a pressure spring element (44) is positioned between the base body (1) of the handle (10) and the end of the claw coupling element (43) of the mechanical coupling (4) of the handle (10) facing away from the engine interface (2), wherein the input shaft (9) is preferably conducted through the pressure spring element (44).

4. A microsurgical instrument (100) according to claim 3,
**characterized in that**
a friction washer (45) is positioned between the pressure spring element (44) and the claw coupling element (43) or one friction washer (45) each is positioned both between the pressure spring element (44) and the claw coupling element (43) and between the pressure spring element (44) and the base body (1), each friction washer being made preferably of a material that comprises a low sliding friction coefficient in friction pairing with the surfaces of the pressure spring element (44) and of the claw coupling element (43), and especially preferably is made of a synthetic.

5. A microsurgical instrument (100) according to at least one of claims 3 or 4,
**characterized in that**
the mechanical coupling (4) comprises a coupling housing (41) that is connected with the base body (1) and in which the claw coupling element (43) is enclosed,
- wherein the coupling housing (41), on a free end facing the engine interface (2), comprises an opening (413), through which a predetermined claw coupling element (52) of the engine block can be conducted, and
- wherein the pressure spring element (44) preferably is supported on an end of the coupling housing (41) facing away from the opening (413).

6. A microsurgical instrument (100) according to claim 5,
**characterized in that**
- the coupling housing (41) has a hollow cylindrical shape, preferably an essentially circular-cylindrical shape, and especially preferably comprises on the free end at least one rounded, tapered or chamfered outer edge (411),
- and wherein a longitudinal axis of the coupling housing (41) and a longitudinal axis of the input shaft (9) are aligned with one another.

7. A microsurgical instrument (100) according to claim 6,
**characterized in that**
the coupling housing (41) on its sheath surface comprises at least one recess (412), preferably three or more recesses (412), which are distributed over the periphery of the coupling housing (41), wherein the recesses (412) are preferably situated in an area of the claw coupling element (43).

8. A microsurgical instrument (100) according to at least one of claims 1 to 7,
**characterized in that**
- the claw coupling element (43) of the mechanical coupling (4) of the handle (10) is connected with the input shaft (9) by a form-fitted shaft-hub connection,
- wherein the input shaft (9) preferably has an external profile, preferably a spline shaft profile, a polygonal profile or one or more flattenings (91) that extend especially preferably along a predetermined sliding pathway of the claw coupling element (43), and the claw coupling element (43) has an internal profile corresponding with the external profile of the input shaft (9).

9. A microsurgical instrument (100) according to at least one of claims 1 to 8,
**characterized in that**
- the handle (10) comprises a gear unit, preferably a bevel gear, which comprises an input bevel gear (92) powered by the input shaft (9) and an output bevel gear (93) that engages with the input bevel gear and drives a secondary shaft, which preferably runs perpendicular to the input shaft (9),
and/or
- the at least one electric contact (3) is situated in a socket or plug, which comprises a contact housing and at least one contact blade (31), wherein the contact housing is connected with the base body (1).

10. A microsurgical instrument (100) according to at least one of claims 1 to 9,
**characterized in that**
the engine interface (2) comprises at least one guide track (21), in which at least one corresponding guide body of the engine block (5) is conducted, wherein the guide track (21) preferably runs parallel to the input shaft (9) in at least one section, especially preferably in an end section facing the mechanical coupling (4).

11. A microsurgical instrument (100) according to at least one of claims 1 to 10,
**characterized in that**
the microsurgical instrument (100) is an electrosurgical microsurgical instrument (100) and the handle (10) comprises an electric actuation element by which a current flow can be activated and deactivated from a power source, preferably a HF-voltage source, to the work piece (20).

12. A microsurgical instrument (100) according to at least one of claims 1 to 11,
**characterized in that**
the claw coupling element (53) of the engine is connected non-rotatably with an engine output shaft (52) and is coupled with the claw coupling element (43) of the mechanical coupling (4), wherein the engine output shaft (52) of the engine block (5) and the input shaft (9) of the handle (10) are aligned in coupled position.

13. A microsurgical instrument (100) according to claim 12,
**characterized in that**
the claw coupling element (53) of the engine block (5) comprises on its periphery at least two claw extensions (531), preferably three or more peripherally distributed claw extensions (531), which extend in the longitudinal direction of the engine output shaft (52) and are distributed peripherally at equal or different angle distances, wherein the claw extensions (531) taper in thickness toward their free ends (533) that point toward the claw coupling element (43) of the mechanical coupling (4).

14. A handle (10) for a microsurgical instrument according to at least one of claims 1 to 13, comprising an engine block (5),
wherein the handle (10) can be coupled with a work piece (20) and comprises:
- a base body (1) and
- an input shaft (9) mounted in the base body (1) and configured for motorized actuation of at least one degree of freedom of the work piece (20), and
- an engine interface (2), into which an engine block (5) can be installed,
wherein at least an electric contact (3) and a mechanical coupling (4) are present on the engine interface (2), wherein the mechanical coupling (4) comprises a claw coupling element (43), which is connected non-rotatably with the input shaft (9) and with which a corresponding claw coupling element (53) of the engine block can be coupled, and
- at least one actuation element, with which a current flow can be activated and deactivated through the electric contact (3) of the engine interface (2),
**characterized in that**
the claw coupling element (43) of the handle (10) is mounted so that it can slide longitudinally on the input shaft (9), wherein the claw coupling element (53) of the engine block (5) comprises on its periphery at least two claw extensions (531) extending in the longitudinal direction of the engine output shaft (52), wherein the thickness of the claw extensions (531) tapers toward their free ends (533).

15. An engine block (5) for a microsurgical instrument (100) according to at least one of claims 1 to 13, wherein the engine block (5)
- can be coupled mechanically and electrically with the engine interface (2) of the handle (10), and
- comprises at least one electric connection element, which can be connected with the electric contact (3) of the handle (10), and
- comprises a claw coupling element (53) that is connected non-rotatably with an engine output shaft (52) and can be coupled with a claw coupling element (43) of the mechanical coupling (4) of the engine interface (2) of the handle (10), wherein the claw coupling element (53) of the engine block (5) comprises at least two peripherally distributed claw extensions (531), which extend in the longitudinal direction of the engine output shaft (52), wherein the thickness of the claw extensions (531) tapers toward their free ends (533).

## Revendications

1. Instrument microchirurgical (100) qui présente une manette (10) et un bloc-moteur (5) pouvant être inséré de manière amovible,
dans lequel la manette (10) peut être accouplée à un insert de travail (20) et présente :
- un corps de base (1),
- un arbre d'entrée (9) logé dans le corps de base (1) pour l'actionnement motorisé d'au moins un degré de liberté de l'insert de travail (20),
- une interface de moteur (2) dans laquelle le bloc-moteur (5) peut être inséré et au niveau de laquelle au moins un contact électrique (3) et un accouplement mécanique (4) sont présents, dans lequel l'accouplement mécanique (4) présente un élément d'accouplement à griffes (43) qui est connecté à l'arbre d'entrée (9) de manière fixe en rotation et peut être accouplé à un élément d'accouplement à griffes (53) côté bloc-moteur correspondant,
- au moins un élément d'actionnement avec lequel un flux de courant peut être activé et désactivé par le contact électrique (3) de l'interface de moteur (2),
**caractérisé en ce que**
l'élément d'accouplement à griffes (43) de la manette (10) est logé sur l'arbre d'entrée (9) de manière coulissante en longueur, dans lequel l'élément d'accouplement à griffes (43) de la manette (10) présente à sa périphérie au moins deux prolongements de griffe (431) qui s'étendent dans la direction longitudinale, qui sont disposés de manière répartie à la périphérie à des écarts angulaires identiques ou différents, dans lequel la largeur des prolongements de griffe (431) rétrécit vers leurs extrémités libres (433) tournées vers l'interface de moteur (2).

2. Instrument microchirurgical (100) selon la revendication 1,
**caractérisé en ce que**
l'élément d'accouplement à griffes (43) de la manette (10) présente à sa périphérie de préférence trois ou plus des prolongements de griffe (431) répartis à la périphérie.

3. Instrument microchirurgical (100) selon la revendication 1 ou 2,
**caractérisé en ce que**
un élément de ressort de compression (44) est disposé entre le corps de base (1) de la manette (10) et l'extrémité de l'élément d'accouplement à griffes (43) de l'accouplement mécanique (4) de la manette (10) détournée de l'interface de moteur (2), dans lequel l'arbre d'entrée (9) est de préférence guidé à travers l'élément de ressort de compression (44).

4. Instrument microchirurgical (100) selon la revendication 3,
**caractérisé en ce que**
un disque de friction (45) est disposé entre l'élément de ressort de compression (44) et l'élément d'accouplement à griffes (43) ou un disque de friction (45) est à chaque fois disposé entre l'élément de ressort de compression (44) et l'élément d'accouplement à griffes (43) et entre l'élément de ressort de compression (44) et le corps de base (1), lequel se compose de préférence d'un matériau qui présente dans un appariement de friction avec les surfaces de l'élément de ressort de compression (44) et l'élément d'accouplement à griffes (43) un faible coefficient de friction de glissement et se compose de manière particulièrement préférée d'un plastique.

5. Instrument microchirurgical (100) selon au moins une des revendications 3 ou 4,
**caractérisé en ce que**
l'accouplement mécanique (4) présente un logement d'accouplement (41) qui est connecté au corps de base (1) et dans lequel est reçu l'élément d'accouplement à griffes (43),
- dans lequel le logement d'accouplement (41) présente à une extrémité libre tournée vers l'interface de moteur (2) une ouverture (413) à travers laquelle un élément d'accouplement à griffes côté bloc-moteur prédéfini (52) peut être guidé, et
- dans lequel l'élément de ressort de compression (44) s'appuie de préférence sur une extrémité du logement d'accouplement (41) détournée de l'ouverture (413).

6. Instrument microchirurgical (100) selon la revendication 5,
**caractérisé en ce que**
- le logement d'accouplement (41) a une forme cylindrique creuse, de préférence une forme essentiellement cylindrique circulaire, et présente de manière particulièrement préférée à l'extrémité libre au moins une arête externe (411) arrondie, biseautée ou chanfreinée,
- et dans lequel un axe longitudinal du logement d'accouplement (41) et un axe longitudinal de l'arbre d'entrée (9) s'alignent.

7. Instrument microchirurgical (100) selon la revendication 6,
**caractérisé en ce que**
le logement d'accouplement (41) présente au niveau de sa face d'enveloppe au moins un évidement (412), de préférence trois évidements (412) ou plus qui sont présents de manière répartie sur la périphérie du logement d'accouplement (41), dans lequel les évidements (412) sont de préférence présents dans une région de l'élément d'accouplement à griffes (43).

8. Instrument microchirurgical (100) selon au moins une des revendications 1 à 7,
**caractérisé en ce que**
- l'élément d'accouplement à griffes (43) de l'accouplement mécanique (4) de la manette (10) est connecté à l'arbre d'entrée (9) par le biais d'une connexion arbre-moyeu par conjugaison de formes,
- dans lequel l'arbre d'entrée (9) présente de préférence un profil externe, de préférence un profil d'arbre cannelé, un profil polygonal ou un ou plusieurs aplatissement(s) (91) qui s'étend de manière particulièrement préférée le long d'un trajet de coulissement prédéterminé de l'élément d'accouplement à griffes (43), et l'élément d'accouplement à griffes (43) présente un profil interne correspondant au profil externe de l'arbre d'entrée (9).

9. Instrument microchirurgical (100) selon au moins une des revendications 1 à 8,
**caractérisé en ce que**
- la manette (10) présente un engrenage, de préférence un engrenage à pignons coniques, qui présente un pignon d'attaque (92) entraîné par l'arbre d'entrée (9) et un pignon de sortie (93) s'engrenant avec celui-ci qui entraîne un arbre secondaire qui s'étend de préférence perpendiculairement à l'arbre d'entrée (9),
et/ou
- l'au moins un contact électrique (3) est présent dans une prise femelle ou mâle qui présente un logement de contact et au moins une languette de contact (31), dans lequel le logement de contact est connecté au corps de base (1).

10. Instrument microchirurgical (100) selon au moins une des revendications 1 à 9,
**caractérisé en ce que**
l'interface de moteur (2) présente au moins un rail de guidage (21) dans lequel au moins un corps de guidage correspondant du bloc-moteur (5) est guidé, dans lequel le rail de guidage (21) s'étend de préférence dans au moins une section parallèlement à l'arbre d'entrée (9), de manière particulièrement préférée dans une section d'extrémité tournée vers l'accouplement mécanique (4).

11. Instrument microchirurgical (100) selon au moins une des revendications 1 à 10,
**caractérisé en ce que**
l'instrument microchirurgical (100) est un instrument microchirurgical électrochirurgical (100) et la manette (10) présente un élément d'actionnement électrique par le biais duquel un flux de courant peut être activé et désactivé par une source de tension, de préférence une source de tension RF, vers l'insert de travail (20).

12. Instrument microchirurgical (100) selon au moins une des revendications 1 à 11,
**caractérisé en ce que**
l'élément d'accouplement à griffes côté moteur (53) est connecté à un arbre de sortie moteur (52) de manière fixe en rotation et est accouplé à l'élément d'accouplement à griffes (43) de l'accouplement mécanique (4), dans lequel l'arbre de sortie moteur (52) du bloc-moteur (5) et l'arbre d'entrée (9) de la manette (10) s'alignent dans l'état accouplé.

13. Instrument microchirurgical (100) selon la revendication 12,
**caractérisé en ce que**
l'élément d'accouplement à griffes (53) du bloc-moteur (5) présente à sa périphérie au moins deux prolongements de griffe (531), de préférence trois prolongements de griffe (531) répartis à la périphérie ou plus, qui s'étendent dans la direction longitudinale de l'arbre de sortie moteur (52), qui sont disposés de manière répartie à la périphérie à des écarts angulaires identiques ou différents, dans lequel les prolongements de griffe (531) rétrécissent en largeur vers leurs extrémités libres (533) tournées vers l'élément d'accouplement à griffes (43) de l'accouplement mécanique (4).

14. Manette (10) pour un instrument microchirurgical selon au moins une des revendications 1 à 13 qui présente un bloc-moteur (5),
dans laquelle la manette (10) peut être accouplée à un insert de travail (20) et présente :
- un corps de base (1) et
- un arbre d'entrée (9) logé dans le corps de base (1) qui est réalisé pour l'actionnement motorisé d'au moins un degré de liberté de l'insert de travail (20), et
- une interface de moteur (2) dans laquelle un bloc-moteur (5) peut être inséré, dans laquelle au moins un contact électrique (3) et un accouplement mécanique (4) sont présents au niveau de l'interface de moteur (2), dans laquelle l'accouplement mécanique (4) présente un élément d'accouplement à griffes (43) qui est connecté à l'arbre d'entrée (9) de manière fixe en rotation et avec lequel un élément d'accouplement à griffes (53) côté bloc-moteur correspondant peut être accouplé, et
- au moins un élément d'actionnement avec lequel un flux de courant peut être activé et désactivé par le contact électrique (3) de l'interface de moteur (2),
**caractérisée en ce que**
l'élément d'accouplement à griffes (43) de la manette (10) est logé sur l'arbre d'entrée (9) de manière coulissante en longueur, dans laquelle l'élément d'accouplement à griffes (53) du bloc-moteur (5) présente au moins deux prolongements de griffe (531) répartis à la périphérie qui s'étendent dans la direction longitudinale de l'arbre de sortie moteur (52) et dont la largeur rétrécit vers leurs extrémités libres (533).

15. Bloc-moteur (5) pour un instrument microchirurgical (100) selon au moins une des revendications 1 à 13, dans lequel le bloc-moteur (5)
- peut être accouplé mécaniquement et électriquement à l'interface de moteur (2) de la manette (10), et
- présente au moins un élément de connexion électrique qui peut être connecté au contact électrique (3) de la manette (10), et
- présente un élément d'accouplement à griffes (53) qui est connecté à un arbre de sortie moteur (52) de manière fixe en rotation et peut être accouplé à un élément d'accouplement à griffes (43) de l'accouplement mécanique (4) de l'interface de moteur (2) de la manette (10), dans lequel l'élément d'accouplement à griffes (53) du bloc-moteur (5) présente au moins deux prolongements de griffe (531) répartis à la périphérie qui s'étendent dans la direction longitudinale de l'arbre de sortie moteur (52) et dont la largeur rétrécit vers leurs extrémités libres (533).
